(19) Europäisches Patentamt
European Patent Office
Office européen des brevets

(11) **EP 2 287 325 A1**

(12) **EUROPEAN PATENT APPLICATION**
published in accordance with Art. 153(4) EPC

(43) Date of publication:
**23.02.2011 Bulletin 2011/08**

(21) Application number: **09754368.0**

(22) Date of filing: **09.02.2009**

(51) Int Cl.:
*C12P 7/64* (2006.01)

(86) International application number:
**PCT/JP2009/000513**

(87) International publication number:
**WO 2009/144858 (03.12.2009 Gazette 2009/49)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **29.05.2008 JP 2008141071**
**04.08.2008 JP 2008200360**

(71) Applicant: **Kao Corporation**
**Chuo-Ku**
**Tokyo 103-8210 (JP)**

(72) Inventors:
• **KASE, Minoru**
**Kamisu-shi**
**Ibaraki 314-0103 (JP)**
• **KOMATSU, Toshiteru**
**Kamisu-shi**
**Ibaraki 314-0103 (JP)**

(74) Representative: **HOFFMANN EITLE**
**Patent- und Rechtsanwälte**
**Arabellastraße 4**
**81925 München (DE)**

(54) **METHOD FOR PRODUCING FAT OR OIL CONTAINING LARGE AMOUNT OF DIACYLGLYCEROL**

(57)    Provided is a process for producing a diacylglycerol-rich fat or oil efficiently under an industrially advantageous condition. Provided is a process for producing a diacylglycerol-rich fat or oil, including subjecting monoacylglycerols to a transesterification reaction by using a lipase in the presence of water.

**Description**

Field of the Invention

The present invention relates to a process for producing a diacylglycerol-rich fat or oil.

Background of the Invention

A fat or oil containing diacylglycerols at a high concentration has a physiological action such as a body-fat burning action, and hence is widely used as an edible oil. For producing diacylglycerols, generally used is a process utilizing a glycerolysis reaction between glycerin and a fat or oil or a process utilizing an esterification reaction between glycerin and a fatty acid (for example, Patent Documents 1 to 3). There is also used a process in which a monoglyceride and a fat or oil are subjected to a transesterification reaction (Patent Document 4).

Further, there is another process in which a fatty acid used for an esterification reaction is produced by hydrolyzing a raw material fat or oil through a combination of a high-pressure splitting method and an enzymatic splitting method, and the resultant fatty acid is subjected to esterification with glycerin (Patent Document 5). Those production processes are roughly classified into chemical methods using an alkali catalyst or the like and enzymatic methods using an enzyme such as a lipase.

Patent Document 1: JP-A-6-65310
Patent Document 2: JP-A-6-65311
Patent Document 3: JP-A-4-330289
Patent Document 4: JP-A-2000-345189
Patent Document 5: JP-A-2006-137923

Summary of the Invention

The present invention provides a process for producing a diacylglycerol-rich fat or oil, including subjecting monoacylglycerols to a transesterification reaction by using a lipase in the presence of water.

Detailed Description of the Invention

In the conventional techniques described above, when the glycerolysis reaction or the trans esterification reaction is carried out, the resultant reaction product per se contains diacylglycerols in low purity, and hence, for example, a high-vacuum distillation facility is necessary for converting the reaction product to one containing high-purity diacylglycerols. Thus, the conventional techniques have problems in the purity of the diacylglycerols and industrial productivity. Further, when esterification is carried out, the purity of diacylglycerols can be increased depending on the purity of a fatty acid used as a reaction raw material or on condition setting. However, such process is not necessarily satisfactory in the balance between production efficiency and quality and in cost.

Thus, the present invention relates to providing a process for efficiently producing a diacylglycerol-rich fat or oil under an industrially advantageous condition.

The inventors of the present invention have conducted extensive investigations on a process for producing diacylglycerols, and have found that high-purity diacylglycerols are efficiently obtained by subjecting monoacylglycerols to a transesterification reaction.

According to the production process of the present invention, a diacylglycerol-rich fat or oil can be efficiently obtained without using an expensive raw material or a special facility, and hence the production process is extremely advantageous industrially.

Examples of the monoacylglycerols to be used in the process of the present invention (hereinafter, may also be referred to as "raw material monoacylglycerols") include a monoacylglycerol in which the 1-hydroxyl group of glycerin is esterified with a fatty acid (1-monoacylglycerol), a monoacylglycerol in which the 2-hydroxyl group is esterified with a fatty acid (2-monoacylglycerol), and a monoacylglycerol in which the 3-hydroxyl group is esterified with a fatty acid (3-monoacylglycerol). Preferred monoacylglycerols are those including 1-monoacylglycerol or 3-monoacylglycerol at a high proportion.

The number of carbon atoms in the fatty acid residue of each of the raw material monoacylglycerols is not particularly limited, but is preferably 8 to 24, more preferably 14 to 24, and even more preferably 16 to 22. The fatty acid residue includes saturated and unsaturated ones. Specific examples of the fatty acid residue include acyl groups derived from caprylic acid, capric acid, lauric acid, myristic acid, palmitic acid, stearic acid, oleic acid, linoleic acid, linolenic acid, eicosapentaenoic acid, and docosahexaenoic acid; and acyl groups derived from fatty acids obtained from animal oils containing any of those acids, such as beef tallow and lard, and obtained from plant oils containing any of those acids, such as rapeseed oil, soybean oil, corn oil, sunflower oil, cotton seed oil, *shiso* oil, stillingia oil, linseed oil, safflower oil, perilla oil, and palm oil.

Those raw material monoacylglycerols may be used singly or in combination of two or more species.

The content of unsaturated fatty acids in the total constituent fatty acids in the raw material monoacylglycerols is preferably 50% by mass (hereinafter, simply denoted as "%") or more, more preferably 60% or more, and even more preferably 70% or more, from the viewpoints of the appearance, physiological effect, and industrial productivity of a final product. Further, oleic acid, linoleic acid, and linolenic acid are preferred as the unsaturated fatty acids.

In addition, the content of trans-unsaturated fatty acids in the constituent fatty acids in the raw material monoacylglycerols is preferably 8% or less, and more preferably 4% or less from the viewpoint of reducing the content of trans-unsaturated fatty acids in the final product, and moreover, is preferably 2% or less, more preferably 1.5% or less, even more preferably 1% or less, and even more preferably 0.5% or less from the viewpoint of reducing the content of trans-unsaturated fatty acids in the final product to a higher extent.

The raw material monoacylglycerols may be obtained by any method such as a hydrolysis reaction of a fat or oil such as rapeseed oil, sunflower oil, corn oil, soybean oil, rice oil, safflower oil, cottonseedoil, beef tallow, linseed oil, or fish oil; a glycerolysis reaction between any of those fats or oils and glycerin; or an esterification reaction between a fatty acid derived from any of those fats or oils and glycerin. Further, the fat or oil may also be a separated fat or oil, a mixed fat or oil, or a fat or oil in which the fatty acid composition is adjusted by hydrogenation or a transesterification reaction. A fat or oil which is not hydrogenated is preferred from the viewpoint of reducing the content of trans-unsaturated fatty acids in the constituent fatty acids in the fat or oil. The reaction method may be carried out by a chemical method using an alkali catalyst or the like or an enzymatic method using an enzyme such as a lipase. The resultant reaction product may be subjected to distillation, fractionation, solvent extraction, steam distillation, or the like, to thereby yield desired raw material monoacylglycerols.

It is preferred that a reacted oil (hereinafter, may also be referred to as "glycerolysis reacted oil") obtained by a glycerolysis reaction between a fat or oil and glycerin be used as the raw material monoacylglycerols from the viewpoints of simplifying a production process, reducing cost, and reducing the content of trans-unsaturated fatty acids in the final product. Further, in a raw material fat or oil to be used in the glycerolysis reaction, the content of trans-unsaturated fatty acids in the constituent fatty acids is preferably 3% or less, more preferably 2% or less, even more preferably 1% or less, and even more preferably 0.5% or less from the viewpoint of reducing the content of trans-unsaturated fatty acids in the final product. To be specific, an undeodorized fat or oil of rapeseed oil, sunflower oil, corn oil, soybean oil, rice oil, safflower oil, cotton seed oil, beef tallow, linseed oil, fish oil, or the like is preferably used. Note that the undeodorized fat or oil in the present invention refers to a fat or oil which has not been subjected to deodorization during a purification treatment of the fat or oil.

Any conventionally known method may be adopted as a method of performing a glycerolysis reaction between a fat or oil and glycerin, and any one of the chemical method and the enzymatic method can be adopted. The glycerolysis reaction by the chemical method is preferred from the viewpoint of reducing the content of triacylglycerols in the final product. The glycerolysis reaction by the enzymatic method is preferred from the viewpoint of preventing the increase of the content of trans-unsaturated fatty acids in the final product. A lipase is preferably used as an enzyme. Examples of the lipase include lipases as used for transesterification reactions. When the glycerolysis reaction is carried out by the enzymatic method, the reaction temperature is preferably 0 to 100°C, more preferably 20 to 80°C, and even more preferably 30 to 80°C from the viewpoints of, for example, enhancing the reaction rate and inhibiting the inactivation of the lipase.

Meanwhile, when the glycerolysis reaction is carried out by the chemical method, it is preferred to use, as a catalyst, an alkali such as sodium hydroxide or calcium hydroxide, or an acid such as an organic acid or a salt thereof, from the viewpoints of enhancing the reaction rate and improving the color of a reaction oil. The reaction temperature is preferably 100 to 300°C and more preferably 150 to 250°C from the viewpoints of enhancing the reaction rate and inhibiting the production of trans-unsaturated fatty acids.

The raw material monoacylglycerols in the present invention may contain monoacylglycerols, diacylglycerols, triacylglycerols, and glycerin. In an embodiment of the present invention, the content of glycerin is preferably 15% or less, more preferably 10% or less, even more preferably 0 to 5%, even more preferably 0 to 3%, and even more preferably 0.1 to 1% from the viewpoints of increasing the content of the diacylglycerols and reducing the burden of raw material purification. Note that when a glycerolysis reacted oil obtained by a glycerolysis reaction between a fat or oil and glycerin is used as the rawmaterial monoacylglycerols, glycerin is preferably removed from the glycerolysis reacted oil before use.

Examples of a method of removing glycerin include a method of removing a glycerin layer by centrifugal separation, a method of distilling glycerin under reduced pressure, a method of removing glycerin by steam distillation, a method of removing a glycerin layer by phase separation, a removing method by water washing, and a removing method by using an adsorbent or the like. However, the method of removing glycerin is not limited to those described above and those methods may be used in combination.

The content of the triacylglycerols in the raw material monoacylglycerols is preferably 50% or less, more preferably 40% or less, and even more preferably 0.1 to 30% from the viewpoint of enhancing the purity of diacylglycerols in the final product. Further, the content of the diacylglycerols in the raw material monoacylglycerols is preferably 40% or less, more preferably 30% or less, and even more preferably 0.1 to 20%. The content of fatty acids in the raw material monoacylglycerols is preferably 50% or less, more preferably 40% or less, even more preferably 0.1 to 30%, even more preferably 0.1 to 20%, and even more preferably 0.1 to 10%. The content of the monoacylglycerols in the raw material monoacylglycerols is preferably 45% or more, more preferably 50% or more, and even more preferably 55 to 99%.

As for the lipase used for the transesterification reaction, not only animal-derived lipases and plant-derived lipases, but also commercially available, microorganism-derived lipases, and immobilized lipases produced by immobilizing lipases, may be used. Examples of the lipases used for the transesterification reaction include lipases originated from microorganisms belonging to, for example, the genus *Rizopus,* the genus *Aspergillus,* the genus *Chromobacterium,* the genus *Mucor,* the genus *Rhizomucor,* the genus *Pseudomonas,* the genus *Geotrichum,* the genus *Penicillium,* and the genus *Candida*, and animal lipases such as a pancreatic lipase. In particular, in order to produce a functional fat or oil by the transesterification reaction, lipases originated from microorganisms belonging to the genus *Penicillium,* the genus *Rhizomucor,* the genus *Candida*, and the like are preferred among the lipases originated from microorganisms.

In addition, using an immobilized lipase produced by immobilizing a lipase to a carrier is preferred from the viewpoint of effectively utilizing lipase activity. Further, using the immobilized lipase is preferred also from the viewpoint that the reaction product and the lipase are simply separated.

Examples of the carrier for immobilization include inorganic carriers such as celite, diatomaceous earth, kaolinite, silica gel, molecular sieves, porous glass, activated carbon, calcium carbonate, and ceramics; ceramics powder; and organic polymers such as polyvinyl alcohol, polypropylene, chitosan, an ion-exchange resin, a hydrophobic adsorptive resin, a chelating resin, and a synthetic adsorptive resin. The ion-exchange resin is more preferred from the viewpoint of its high water-retaining power. Further, the ion-exchange resin is preferably porous from the viewpoint of having a large surface area and thereby being capable of increasing the adsorption amount of lipase.

The particle size of the resin to be used as the carrier for immobilization is preferably 50 to 2000 $\mu$m and more preferably 100 to 1000 $\mu$m. The pore diameter is preferably 10 to 150 nm and more preferably 10 to 100 nm. Examples of the raw material of the resin include a phenol formaldehyde-based resin, a polystyrene-based resin, an acrylamide-based resin, and a divinylbenzene-based resin. The phenol formaldehyde-based resin (for example, Duolite A-568 manufactured by Rohm and Hass) is more preferred from the view point of enhancing lipase adsorption property.

When the lipase is immobilized, the lipase may be adsorbed directly to the carrier for immobilization, or the carrier may be preliminarily treated with a lipophilic fatty acid or a derivative thereof before the lipase adsorption in order to attain such an adsorption state that allows the lipase to express high activity. Examples of the lipophilic fatty acid to be used include saturated or unsaturated, linear or branched fatty acids having 8 to 18 carbon atoms, which may be substituted with a hydroxyl group. Specific examples thereof include capric acid, lauric acid, myristic acid, oleic acid, linoleic acid, $\alpha$-linolenic acid, ricinoleic acid, and isostearic acid. Examples of the derivatives of those fatty acids include esters of those fatty acids and a monohydric or polyhydric alcohol, phospholipids, and derivatives in each of which an ethylene oxide is added to any of those esters. Specific examples thereof include methyl esters, ethyl esters, monoglycerides, and diglycerides of any of the above-mentioned fatty acids, ethylene oxide adducts thereof, polyglycerin esters, sorbitan esters, and sucrose esters. Those lipophilic fatty acids or derivatives thereof may be used in combination of two or more species.

A method of bringing any of those lipophilic fatty acids or derivatives thereof into contact with the carrier may include adding any of those lipophilic fatty acids or derivatives thereof directly to the carrier in water or an organic solvent. However, in order to improve dispersibility, any of those lipophilic fatty acids or derivatives thereof maybe first dispersed and dissolved in an organic solvent, and then the resultant maybe added to the carrier dispersed in water. Examples of the organic solvent include chloroform, hexane, acetone, and ethanol. The amount of use of the lipophilic fatty acids or derivatives thereof is preferably 1 to 1000 parts by mass (hereinafter, simply denoted as "parts") and more preferably 10 to 500 parts with respect to 100 parts of the carrier. The contact temperature is preferably 0 to 80°C and more preferably 20 to 60°C. The contact time is preferably about 5 minutes to 5 hours. After the carrier is subjected to the above treatment, the carrier is collected through filtration, and then may be dried. The drying temperature is preferably 0 to 80°C. Drying under reduced pressure may be carried out.

The temperature at which the immobilization of the lipase is carried out can be determined depending on the characteristics of the lipase, but the temperature is a temperature at which the inactivation of the lipase does not occur, that is, preferably 0 to 80°C and more preferably 20 to 60°C. Further, the pH of a lipase solution which is used at the time of the immobilization may only fall within the range at which denature of the lipase does not occur, and hence the pH can be determined depending on the characteristics of the lipase in the same way as for the temperature, but the pH is preferably 3 to 9. In order to keep the pH level, a buffer solution is used. Examples of the buffer solution include an acetate buffer solution, a phosphate buffer solution, and a Tris-hydrochloric acid buffer solution. The concentration of the lipase in the above-mentioned lipase solution is preferably equal to or lower than the saturation solubility of the lipase and a sufficient concentration from the viewpoint of immobilization efficiency. Further, as the lipase solution, a supernatant obtained by removing an insoluble portion by centrifugal separation or a lipase solution purified by ultrafiltration or the like may be used as required. Further, the mass of the lipase to be used varies depending on the lipase activity, but is preferably 5 to 1000 parts and more preferably 10 to 500 parts with respect to 100 parts of the carrier.

From the viewpoint that the state of the immobilized lipase is converted to that suitable for the transesterification reaction, it is preferred that the immobilized lipase be recovered from the lipase solution by filtration, followed by removal of excess water, and the resultant immobilized lipase be brought into contact with a raw material serving as a reaction substrate without being dried. The content of water in the immobilized lipase after the contact varies depending on the type of the carrier that is used, but is preferably 0.1 to 100 parts, more preferably 0.5 to 50 parts, and even more preferably 1 to 30 parts, with respect to 100 parts of the carrier for immobilization. In this case, the immobilized lipase may be encapsulated in a filling container such as a column and the raw material may be circulated by using a pump or the like, or the immobilized lipase may be dispersed in the raw material. The contact temperature is preferably 0 to 80°C and can be selected depending on the characteristics of the lipase. Moreover, the contact time is preferably about 0.2 to 200 hours and more preferably 3 hours to 100 hours from the viewpoint of industrial productivity. The immobilized lipase is preferably recovered by filtration after the completion of the contact from the viewpoint of industrial productivity.

The transesterification activity of the immobilized lipase is preferably such that when a transesterification reaction is carried out under the same conditions as those in the method described in Test Example 1 mentioned below except that the reaction time is 3 hours, the content of diacylglycerols in a reaction product increases compared with the content of diacylglycerols in a reaction raw material by preferably 1% or more, more preferably 3% or more, and even more preferably 10% or more.

In the present invention, the transesterification reaction of monoacylglycerols can be carried out in a batch mode, a continuous mode, or a semi-continuous mode. A raw material to be supplied into a reaction device is preferably subjected to preliminary dehydration, deaeration, or deoxidation under reduced pressure from the viewpoints of increasing the content of diacylglycerols in the resultant reaction product and inhibiting the deterioration of the resultant reaction product.

The amount of the lipase to be used in the reaction can be appropriately determined by taking the activity of the lipase into consideration, but is preferably 0.01 to 100 parts, more preferably 0.1 to 50 parts, and even more preferably 0.2 to 30 parts with respect to 100 parts of the raw material monoacylglycerols to be degraded by the lipase.

The transesterification reaction is carried out in the presence of water. The water content in a reaction system is set to preferably 4% or less, more preferably 0.01 to 3%, and even more preferably 0.02 to 2% from the viewpoint of increasing the content of diacylglycerols in the reaction product. Any of distilled water, ion-exchanged water, tap water, well water, and the like may be used as the water. Further, glycerin or any other water-soluble components may be mixed in the water. A buffer solution having a pH of 3 to 9 may be used as required so as to maintain the stability of the lipase. The water maybe water included in the lipase or the raw material monoacylglycerols. It is preferred that the total water content be controlled at 4% or less. Examples of a method of controlling the total water content include (i) a method of controlling the total water content by preliminarily measuring the water content in each component by the Karl Fischer method or the like, and (ii) a method in which the reaction components are completely dehydrated and then a predetermined amount of water is added. The method (i) is preferred because handling of a hygroscopic product such as a powdered lipase is simple. Note that the content of water retained in the immobilized lipase is also included in the above-mentioned water content.

The reaction temperature is preferably 0 to 80°C and more preferably 20 to 70°C, which is a temperature at which the activity of the lipase is exhibited more effectively and free fatty acids produced by degradation do not form crystals. Further, the reaction is preferably carried out in the presence of an inert gas such as nitrogen so as to avoid the contact with air as far as possible.

The reaction may be carried out in the presence of a solvent such as hexane, cyclohexane, or petroleum ether.

In the present invention, the purity of diacylglycerols in the reaction product is preferably 50% or more, more preferably 60 to 99%, even more preferably 70 to 98%, and even more preferably 80 to 97% from the viewpoints of a physiological effect and industrial productivity. Here, the purity of diacylglycerols is calculated based on "diacylglycerols/(diacylglycerols+triacylglicerols) $\times$ 100".

In the present invention, the content of (diacylglycerols+triacylglycerols) (% by mass) in the reaction product is preferably 50% or more, more preferably 50 to 99%, even more preferably 55 to 98%, and even more preferably 60 to 97% from the viewpoints of a physiological effect and industrial productivity.

Further, the content of monoacylglycerols in the reaction product is preferably 2 to 60%, more preferably 3 to 50%, even more preferably 5 to 50%, and even more preferably 10 to 40% from the viewpoints of reducing the burden of distillation and improving reaction efficiency.

The reaction product obtained by the transesterification reaction using the lipase according to the process of the present invention is high in the purity of diacylglycerols, and hence is useful as a fat or oil having a high physiological effect.

A diacylglycerol-rich fat or oil obtained by the transesterification reaction can be converted to a product by being subjected to a post-treatment. Respective steps of distillation, acid treatment, water washing, and deodorization are preferably carried out as the post-treatment.

The distillation step refers to a step for removing fatty acids produced as by-products and unreacted monoacylglycerols from the reaction product by subjecting the diacylglycerol-rich fat or oil obtained by the transesterification reaction to distillation under reduced pressure.

In recent years, social demands for environmental problems have been increasing. A production technology that can strike a balance between the production of a high-quality product and a reduction in environmental load have been strongly desired. In order to reduce the environmental load, reducing the amount of waste substances discharged during the production process is effective. In view of the foregoing, in an embodiment of the present invention, it is preferred that when the diacylglycerol-rich fat or oil is produced, the fatty acids produced as by-products and unreacted monoacylglycerols both removed from the reaction product during the distillation step be recovered as a distillation recovery oil, and the distillation recovery oil be recycled as a part or the whole of the next reaction raw material. In addition, using the distillation recovery oil is preferred also from the viewpoint of increasing the content of diacylglycerols.

For the conditions of the distillation step, the pressure is preferably 1 to 300 Pa, more preferably 1.5 to 200 Pa, and even more preferably 2 to 100 Pa from the viewpoints that facility cost and operation cost are reduced, the distillation ability is increased, the distillation temperature can be optimally selected, and an increase in trans-unsaturated fatty acids or thermal deterioration caused by a thermal history is inhibited. The temperature is preferably 180 to 280°C, more preferably 190 to 260°C, and even more preferably 200 to 250°C from the viewpoint of inhibiting the trans-unsaturated fatty acids from increasing. The retention time is preferably 0.2 to 30 minutes, more preferably 0.2 to 20 minutes, and even more preferably 0.2 to 10 minutes from the viewpoint of inhibiting the trans-unsaturated fatty acids from increasing. Here, the retention time refers to an average retention time taken during the period in which the temperature of a fat or oil reaches the distillation temperature.

The acid treatment step refers to a step in which a chelating agent such as citric acid is added to and mixed with the distillate oil, followed by dehydration under reduced pressure. Further, the resultant acid-treated oil may be subjected to a decolorization step based on contacting with an adsorbent from the viewpoint of further improving the color and flavor. The water-washing step refers to a step in which water is added to the acid-treated oil, followed by vigorously stirring, to thereby carry out oil-water separation. The water washing is preferably repeated a plurality of times (for example, three times) to yield a water-washed oil.

The deodorization treatment is basically performed by steam distillation under reduced pressure. Examples of the steam distillation under reduced pressure include a batch mode, a semi-continuous mode, and a continuous mode. When the amount of the fat or oil is small, the batch mode is preferably used, and when the amount is large, the semi-continuous mode or the continuous mode is preferably used. Examples of the apparatus for the semi-continuous mode include a tray type deodorization apparatus (Girdler type deodorization apparatus) constructed from a deodorization tower provided with several stacks of trays. In the apparatus, a fat or oil to be deodorized is supplied from the top, and the oil successively moves intermittently down to lower trays, resulting in its deodorization. Examples of the apparatus for the continuous mode include a thin layer deodorization apparatus in which the deodorization tower is packed with a structure that is well balanced between the gas-liquid contact efficiency and a low pressure loss, and thus the efficiency of contact with steam has been enhanced.

The deodorization treatment can be performed by a method of performing the treatment using either the thin layer deodorization apparatus or the tray type deodorization apparatus alone, or a method of performing the treatment by combining the deodorization treatment using the thin layer deodorization apparatus and the deodorization treatment using the tray type deodorization apparatus. In the case of the present invention, the method of performing the deodorization treatment using either the thin layer deodorization apparatus or the tray type deodorization apparatus alone is preferred from the viewpoints of apparatus cost, the content of trans-unsaturated fatty acids, and the flavor characteristic to diacylglycerols.

In general, when a deodorization treatment is performed, the odorous components contained in a water-washed oil are removed, carotenoid-based pigments become light-colored because of thermal degradation, and moreover, the impurities contained therein in a trace amount become inert, yielding a stable substance. Thus, when a normal fat or oil is subjected to deodorization, stricter conditions yields a fat or oil which has a preferred flavor. However, in the diacylglycerol-rich fat or oil, the deodorization step affects its rich body taste, and hence the quality of a product depends on the conditions of the deodorization treatment.

In the present invention, the deodorization treatment is carried out so that a deodorization time (x [minute]) and a deodorization temperature (y [°C]) fall within the ranges which satisfy the following equation (i).

$$350 \leq (y-210) \times x \leq 2100$$

(where 215≤y≤280)

When the deodorization treatment is carried out at a thermal history lower than the range defined by the equation (i), or when the deodorization treatment is carried out at a deodorization temperature (y) lower than 215°C, a fat or oil in which a sensation of stimulation and heaviness are reduced may not be obtained. When the deodorization treatment is carried out at a thermal history higher than the range def ined by the equation (i) or at a deodorization temperature (y) higher than 280°C, a fat or oil which is excellent in a rich body taste may not be obtained, and an inhibiting effect on the increase in trans-unsaturated fatty acids is not sufficiently exhibited. Here, x represents a deodorization time (minutes), and y represents a deodorization temperature (°C). Note that in the case where temperatures change time-dependently during deodorization, the deodorization temperature is an average of the temperatures. Further, from the viewpoints of deodorization efficiency, improving a flavor, and inhibiting the increase in trans-unsaturated fatty acids, the deodorization treatment is carried out more preferably within the range satisfying the following equation (ii)

$$400 < (y-210) \times x < 1900,$$

even more preferably within the range satisfying the following equation (iii)

$$450 < (y-210) \times x < 1700,$$

and
even more preferably within the range satisfying the following equation (iv)

$$500 < (y-210) \times x < 1600.$$

Note that any one of the ranges satisfies 215≤y≤280.

In the present invention, as the deodorization treatment is carried out under the condition defined by the above-mentioned equation (i), the deodorization time changes depending on the deodorization temperature. To be specific, when the deodorization treatment is carried out at 250 to 270°C, the deodorization time is preferably set to 6 to 35 minutes. When the deodorization treatment is carried out at 235 to 250°C, the deodorization time is preferably set to 9 to 53 minutes. When the deodorization treatment is carried out at 220 to 235°C, the deodorization time is preferably set to 14 to 120 minutes.

Steam distillation under reduced pressure is preferred as the deodorization treatment. The amount of steam to be used is set to preferably 0.3 to 20% and more preferably 0.5 to 10% with respect to the fat or oil from the viewpoint of improving the flavor such as "umami taste" and "rich body taste" characteristic to diacylglycerols. Further, the pressure is set to preferably 0.01 to 4 kPa and more preferably 0.06 to 0.6 kPa from the same viewpoint as described above. In particular, when setting the deodorization temperature to 255 to 280°C, the amount of steam is set to preferably 0.3 to 3%, more preferably 0.4 to 2.5%, and even more preferably 0.5 to 2.2% with respect to the fat or oil from the viewpoint of improving the flavor such as "umami taste" and "rich body taste" characteristic to diacylglycerols. Further, when setting the deodorization temperature to 250 to 255°C, the amount of steam is set to preferably 2.1 to 5%, more preferably 2.2 to 4.5%, and even more preferably 2.5 to 4% with respect to the fat or oil from the same viewpoint as that described above. Even further, when setting the deodorization temperature to 215 to 250°C, the amount of steam is set to preferably 2.1 to 10%, more preferably 2.2 to 8%, and even more preferably 2.5 to 6% with respect to the fat or oil from the same viewpoint as that described above.

The heatup time to the deodorization temperature is preferably set to 0.5 to 60 minutes to heat from the temperature 70°C to the temperature 200°C and 0.5 to 45 minutes to heat from the temperature 200°C to the deodorization temperature, more preferably set to 1 to 30 minutes to heat from the temperature 70°C to the temperature 200°C and 1 to 20 minutes to heat from the temperature 200°C to the deodorization temperature, and even more preferably set to 2 to 20 minutes to heat from the temperature 70°C to the temperature 200°C and 2 to 15 minutes to heat from the temperature 200°C to the deodorization temperature, from the viewpoint of inhibiting trans-unsaturated fatty acids from increasing. The cooling time from the deodorization temperature is preferably set to 0.2 to 35 minutes to cool from the deodorization temperature to the temperature 200°C and 0.2 to 40 minutes to cool from the temperature 200°C to the temperature 70°C, more preferably set to 0.5 to 25 minutes to cool from the deodorization temperature to the temperature 200°C and 0.5 to 30 minutes to cool from the temperature 200°C to the temperature 70°C, and even more preferably set to 1 to 20 minutes to cool from the deodorization temperature to the temperature 200°C and 1 to 25 minutes to cool from the temperature 200°C to the temperature 70°C, from the viewpoint of inhibiting trans-unsaturated fatty acids from increasing.

As the result of the deodorization treatment, the increment in the amount of trans-unsaturated fatty acids during a purification step can be controlled to 1% or less, and hence a diacylglycerol-rich fat or oil in which the content of trans-unsaturated fatty acids in the total fatty acids constituting the fat or oil is as small as 2% or less, can be obtained. The content of the trans-unsaturated fatty acids in the diacylglycerol-rich fat or oil is more preferably 0 to 1.5% and even more preferably 0.1 to 1.2% from the viewpoint of a physiological effect.

The post-treatment described above removes unreacted substances and by-products except diacylglycerols and triacylglycerols. Thus, the diacylglycerol-rich fat or oil produced by the process of the present invention has a good flavor and a good color. Further, trans-unsaturated fatty acids are produced in a small amount and the content of diacylglycerols is large, and hence the diacylglycerol-rich fat or oil is useful as a fat or oil having a high physiological effect. A product of the diacylglycerol-rich fat or oil preferably contains diacylglycerols within the range of the "purity of diacylglycerols."

Examples

[Analysis method]

(i) Measurement of glyceride composition

About 3 g of a sample was collected in a test tube which can be used for centrifugation, a 10-minute centrifugation was carried out at 3000 r/min, and precipitated glycerin was removed. Next, about 10 mg of the upper layer and 0.5 mL of a trimethylsilylating agent ("Silylating Agent TH", manufactured by Kanto Chemical Co., Inc.) were loaded into a glass sample bottle, followed by hermetical sealing, and the glass sample bottle was heated at 70°C for 15 minutes. After that, 1.5 mL of water and 1.5 mL of hexane were added, followed by shaking. The whole was left to stand still, and then the upper layer was subjected to gas chromatography (GLC) to analyze glyceride composition.

(ii) Content of trans-unsaturated fatty acids in constituent fatty acids

In accordance with "Method for preparing fatty acid methyl esters (2. 4. 1. 2 - 1996)" in the "Standard Methods for the Analysis of Fats, Oils and Related Materials" edited by Japan Oil Chemists' Society, fatty acid methyl esters were prepared. The resultant samples were measured by the American Oil Chemists' Society Of f icial Method Ce 1f-96 (GLC method).

(iii) Water content in reaction system

The water content in an immobilized lipase or a non-immobilized powdered lipase and the water content in raw material monoacylglycerols were measured by using AQUACOUNTER AQ-7 (HIRANUMA SANGYO Co., Ltd.). Based on the resultant water contents and the amount of use of each raw material, the water content in each reaction system was determined.

(IV) Color

The color of a deodorized oil refers to a value obtained by measuring the deodorized oil by the American Oil Chemists' Society Official Method Ca 13e-92 (Lovibond method) with a 5.25 inch cell and performing calculation based on the following equation (1).

$$\text{Color C} = 10R + Y \qquad (1)$$

(wherein R=Red value and Y=Yellow value)

[Preparation of immobilized lipase]

<Immobilized Lipase G>

500g of Duolite A-568 (manufactured by Rohm & Hass) were stirred in 5000 mL of a 0.1 N aqueous solution of sodium hydroxide for 1 hour. After that, the resultant was washed with 5000 mL of distilled water for 1 hour, and was subjected to pH equilibration with 5000 mL of a 500 mM acetate buffer (pH 5) for 2 hours. After that, pH equilibration was further performed twice each for 2 hours each with 5000 mL of a 50 mM acetate buffer (pH 5). Then, the resultant was filtrated to recover a carrier. After that, 2500 mL of ethanol was used to carry out ethanol substitution for 30 minutes. Then, the resultant was filtrated, 2500 mL of ethanol containing 500 g of a soybean fatty acid were added, and the soybean fatty acid was caused to adsorb to the carrier for 30 minutes. Then, the resultant was filtrated, and the carrier was recovered and washed with 2500 mL of a 50 mM acetate buffer (pH 5) four times to remove ethanol. The resultant was filtrated to recover the carrier. After that, the carrier was brought into contact for 2 hours with 10,000 mL of a 10% solution of a commercially available lipase (Lipase G "Amano" 50, Amano Enzyme Inc. ) for acting on a fat or oil, to thereby carry out immobilization. Further, the resultant was filtrated to recover an immobilized lipase. The immobilized lipase was washed with 2500 mL of a 50 mM acetate buffer (pH 5) to remove a non-immobilized lipase and a protein. Any one of the above operations was performed at a temperature of 20°C. After that, 2000 g of a soybean fatty acid were added and the mixture was subjected to pressure reduction until the pressure reached 400 Pa while being stirred at a temperature of 40°C, thereby being dehydrated. After that, the resultant was stirred in 2500 mL of hexane for 30 minutes, and then an operation for filtrating out a hexane phase was carried out three times. Then, the hexane phase was subjected to solvent removal for 1 hour by using an evaporator at a temperature of 40°C. Next, the resultant was dried under reduced pressure for 15 hours under the conditions of a temperature of 40°C and a pressure of 1300 Pa to perform solvent removal, thereby yielding Immobilized Lipase G.

<Immobilized Lipase AY>

500gofDuoliteA-568 (manufacturedbyRohm&Hass) were stirred in 5000 mL of a 0-1 N aqueous solution of sodium hydroxide for 1 hour. After that, the resultant was washed with 5000 mL of distilled water for 1 hour, and was subjected to pH equilibration with 5000 mL of a 500 mM phosphate buffer (pH 7) for 2 hours. After that, pH equilibration was further performed twice each for 2 hours each with 5000 mL of a 50 mM phosphate buffer (pH 7). Then, the resultant was filtrated to recover a carrier. After that, 2500 mL of ethanol was used to carry out ethanol substitution for 30 minutes. Then, the resultant was filtrated, 2500 mL of ethanol containing 500 g of a soybean fatty acid were added, and the soybean fatty acid was caused to adsorb to the carrier for 30 minutes. Then, the resultant was filtrated, and the carrier was recovered and washed with 2500 mL of a 50 mM phosphate buffer (pH 7) four times to remove ethanol. The resultant was filtrated to recover the carrier. After that, the carrier was brought into contact for 4 hours with 10,000 mL of a 10% solution of a commercially available lipase (Lipase AY "Amano" 30G, Amano Enzyme Inc.) for acting on a fat or oil, to thereby carry out immobilization. Further, the resultant was filtrated to recover an immobilized lipase. The immobilized lipase was washed with 2500 mL of a 50 mM phosphate buffer (pH 7) to remove a non-immobilizedlipaseandaprotein. Anyone of the above operations was performed at a temperature of 20°C. After that, 2000 g of a deodorized soybean oil were added and the mixture was stirred at a temperature of 40°C for 10 hours, followed by filtration, to thereby separate the immobilized lipase from the deodorized soybean oil. After that, the resultant was stirred in 2500 mL of hexane for 30 minutes, and then an operation for filtrating out a hexane phase was carried out three times. Then, the hexane phase was subjected to solvent removal for 1 hour by using an evaporator at a temperature of 40°C. Next, the resultant was dried under reduced pressure for 15 hours under the conditions of a temperature of 40°C and a pressure of 1300 Pa to perform solvent removal, thereby yielding Immobilized Lipase AY.

[Preparation of raw material fat or oil 1]

O-95R (Kao Corporation, the same in the following) was used as a monoacylglycerol serving as a raw material for a transesterification reaction (Material A). In addition, rapeseed oil was added toO-95R, thereby preparing glyceride-mixed oils having different triacylglycerol contents (Materials B, C, D, and E). Further, Healthy Econa Cooking Oil (Kao Corporation, the same in the following) was added to O-95R, thereby preparing glyceride-mixed oils having different diacylglycerol contents (Materials F and G). In addition, rapeseed oil and Healthy Econa Cooking Oil were added to O-95R, thereby preparing a glyceride-mixed oil containing monoacylglycerols, diacylglycerols, and triacylglycerols in nearly the same amount (Material H). Table 1 shows the glyceride composition of each raw material fat or oil.

[Preparation of raw material fat or oil 2]

1000 g of undeodorized rapeseed oil and 343 g of glycerin were loaded into a 2-L four-necked flask equipped with a stirring blade (90 mm × 24 mm). While the mixture was being stirred under a stirring condition of 500 r/min, 0.134 g of calcium hydroxide was added as a catalyst. Next, a glycerolysis reaction was carried out under the condition of a temperature of 210°C and a reaction time of 1 hour while a nitrogen gas was being supplied. After the whole was cooled to 100°C or less, 0.158 g of phosphoric acid was added to neutralize the catalyst. Next, the resultant was cooled to 25°C and was subjected to 10-minute centrifugation at 6000 r/min. The separated glycerin was removed to yield Material I as a raw material monoacylglycerol. Table 1 shows the glyceride compositions of the undeodorized rapeseed oil and Material I. The contents of trans-unsaturated fatty acids in the undeodorized rapeseed oil and Material I were 0.1% and 0.2%, respectively.

[Preparation of raw material fat or oil 3]

2500 g of the above-mentioned undeodorized rapeseed oil and 858 g of glycerin were loaded into a 5-L four-necked flask equipped with a stirring blade (90 mm × 24 mm). While the mixture was being stirred under a stirring condition of 500 r/min, 0.335 g of calcium hydroxide was added as a catalyst. Next, a glycerolysis reaction was carried out under the condition of a temperature of 210°C and a reaction time of 1.5 hours while a nitrogen gas was being supplied, and 0.395 g of phosphoric acid was added to neutralize the catalyst. Next, the resultant was cooled to 25°C and was subjected to 10-minute centrifugation at 6000 r/min, and the separated glycerin was removed. Next, an oil phase was loaded into a 5-L four-necked flask and stirred under a stirring condition of 500 r/min. Distillation of glycerin was initiated while temperature and pressure were gradually being raised. As a result, glycerin was completely distilled under the condition of a temperature of 190°C, a pressure of 100 Pa, and a reaction time of 30 minutes. After that, the resultant was cooled to 70°C to yield Material J as a raw material monoacylglycerol. Table 1 shows the glyceride composition of Material J. The content of trans-unsaturated fatty acids in Material J was 0.2%.

[Table 1]

| [Composition of raw material fat or oil for transesterification] | | | | | | |
|---|---|---|---|---|---|---|
| | Fatty acid [% by mass] | GLY [% by mass] | MAG [% by mass] | DAG [% by mass] | TAG [% by mass] | DAG+TAG Content [% by mass] |
| Material A | 0.9 | 0.8 | 92.1 | 6.3 | 0.0 | 6.3 |
| Material B | 0.6 | 0.7 | 84.7 | 5.4 | 8.5 | 14.0 |
| Material C | 0.6 | 0.6 | 73.4 | 4.9 | 20.5 | 25.4 |
| Material D | 0.5 | 0.5 | 57.4 | 4.2 | 37.4 | 41.7 |
| Material E | 0.4 | 0.4 | 42.2 | 3.4 | 53.7 | 57.1 |
| Material F | 0.7 | 0.6 | 76.8 | 20.5 | 1.5 | 22.0 |
| Material G | 0.6 | 0.3 | 40.1 | 52.6 | 6.4 | 59.0 |
| Material H | 0.4 | 0.2 | 31.7 | 33.0 | 34.7 | 67.7 |
| Material I | 0.1 | 5.1 | 50.9 | 39.9 | 4.1 | 44.0 |
| Material J | 0.1 | 0.0 | 55.7 | 38.7 | 5.5 | 44.2 |
| Distillation recovery oil | 1.8 | 6.4 | 85.3 | 6.5 | 0.0 | 6.5 |

(continued)

| [Composition of raw material fat or oil for transesterification] | | | | | | |
|---|---|---|---|---|---|---|
| | Fatty acid [% by mass] | GLY [% by mass] | MAG [% by mass] | DAG [% by mass] | TAG [% by mass] | DAG+TAG Content [% by mass] |
| Undeodorized rapeseed oil | 0.1 | 0.0 | 0.0 | 1.3 | 98.7 | 99.9 |

GLY: Glycerin
MAG: Monoacylglycerol
DAG: Diacylglycerol
TAG: Triacylglycerol

[Influence of kind of lipase]

Test Example 1

250 g of Material A were loaded into a 500-ML four-necked flask equipped with a stirring blade (75 mm × 20 mm). The flask was left to stand still at 50°C for 30 minutes to stabilize Material A in the flask at 50°C. Next, while Material A was being stirred under the condition of a temperature of 50°C and a stirring of 300 r/min, Immobilized Lipase G (water content: 2.1%) was added at 5% (12.5 g) with respect to Material A, and a transesterification reaction was started. Nitrogen sealing was performed immediately, to thereby convert to a nitrogen atmosphere. When the transesterification reaction was carried out for 50 hours, the glyceride composition reached equilibrium. Then, Immobilized Lipase G was filtrated off, yielding Sample A.

Test Example 2

A transesterification reaction was carried out in the same manner as in Test Example 1 except that a commercially available immobilized lipase, Lipozyme RM IM (Novozymes Japan Ltd. (the same in the following) , water content: 2.2%), was used as an immobilized lipase. When the transesterification reaction was carried out for 67 hours, the glyceride composition reached equilibrium. Then, the immobilized lipase, Lipozyme RM IM, was filtrated off, yielding Sample B.

Test Example 3

A transesterification reaction was carried out in the same manner as in Test Example 1 except that Immobilized Lipase AY (water content: 2.5%) was used as an immobilized lipase. When the transesterification reaction was carried out for 530 hours, the glyceride composition reached equilibrium. Then, Immobilized Lipase AY was filtrated off, yielding Sample C.

Test Example 4

250 g of Material A and 500 ML of hexane were loaded into a 2000-ML four-necked flask equipped with a stirring blade (90 mm × 24 mm) and an air-cooled tube (internal diameter: 11 mm, length: 1 m). The flask was left to stand still at 50°C for 30 minutes to stabilize Material A in the flask at 50°C. Next, while Material A was being stirred under the condition of a temperature of 50°C and a stirring of 300 r/min, Immobilized Lipase G (water content: 2.1%) was added at 5% (12.5 g) with respect to Material A, and a transesterification reaction was started. When the transesterification reaction was carried out for 50 hours, the glyceride composition reached equilibrium. Then, Immobilized Lipase G was filtrated off, yielding Sample D.

Test Example 5

90 g of Material I were loaded into a 200-ML four-necked flask equipped with a stirring blade (50 mm × 18 mm). The flask was left to stand still at 50°C for 30 minutes to stabilize Material I in the flask at 50°C. Next, while Material I was being stirred under the condition of a temperature of 50°C and a stirring of 300 r/min, Immobilized Lipase G (water content: 2.8%) was added at 10% (9.0 g) with respect to Material I, and a transesterification reaction was started. Nitrogen sealing was performed immediately, to thereby convert to a nitrogen atmosphere. The transesterification reaction was carried out for 24 hours. Then, Immobilized Lipase G was filtrated off, yielding Sample U. The content of trans-unsaturated fatty acids in Sample U was 0.2%.

Test Example 6

A transesterification reaction was carried out in the same manner as in Test Example 5 except that a commercially available immobilized lipase, Lipozyme RM IM (water content: 2.6%), was used as an immobilized lipase. The transesterification reaction was carried out for 2 hours. Then, the immobilized lipase, Lipozyme RM IM, was filtrated off, yielding Sample V. The content of trans-unsaturated fatty acids in Sample V was 0.2%.

Table 2 shows the water content in the reaction system and the glyceride composition of the reaction product obtained of each of Test Examples 1 to 6. Note that the glyceride composition refers to a composition of an oil obtained by removing separated glycerin from the reacted oil in accordance with the "analysis method" (the same in the following).

[Table 2]

| | | | Water content in reaction system [% by mass] | Fatty acid [% by mass] | GLY [% by mass] | MAG [% by mass] | DAG [% by mass] | TAG [% by mass] | DAG+TAG Content [% by mass] | DAG Purity [% by mass] |
|---|---|---|---|---|---|---|---|---|---|---|
| [Influence of kind of lipase] | | | | | | | | | | |
| | Reaction product | Reaction raw material | | | | | | | | |
| Test Example 1 | Sample A | Material A | 0.2 | 3.6 | 4.7 | 46.5 | 45.2 | 0.1 | 45.3 | 99.8 |
| Test Example 2 | Sample B | Material A | 0.2 | 2.1 | 2.9 | 31.3 | 63.0 | 0.7 | 63.7 | 98.9 |
| Test Example 3 | Sample C | Material A | 0.2 | 2.9 | 2.6 | 67.2 | 27.3 | 0.0 | 27.3 | 100.0 |
| Test Example 4 | Sample D | material A | 0.2 | 4.8 | 3.3 | 53.4 | 38.4 | 0.1 | 38.4 | 99.8 |
| Test Example 5 | Sample U | Material I | 0.3 | 2.9 | 3.6 | 41.2 | 46.5 | 5.8 | 52.3 | 89.0 |
| Test Example 6 | Sample V | Material I | 0.2 | 1.5 | 2.5 | 33.6 | 54.5 | 7.8 | 62.4 | 87.4 |

[Influence of water content in reaction system]

Test Example 7

250 g of Material A were loaded into a 500-ML four-necked flask equipped with a stirring blade (75 mm × 20 mm). The flask was left to stand still at 50°C for 30 minutes to stabilize Material A in the flask at 50°C. Next, while Material A was being stirred under the condition of a temperature of 50°C and a stirring of 300 r/min, an immobilized lipase, Lipozyme RM IM (water content: 2.5%), was added at 5% (12.5 g) with respect to Material A, and a transesterification reaction was started. Nitrogen sealing was performed immediately, to thereby convert to a nitrogen atmosphere. After the transesterification reaction was carried out for 24 hours, the immobilized lipase, Lipozyme RM IM, was filtrated off, yielding Sample E.

Test Example 8

A transesterification reaction was carried out in the same manner as in Test Example 7 except that distilled water was added at the start of the transesterification reaction so that the water content in the reaction system was set to 1.1%, yielding Sample F.

Test Example 9

A transesterification reaction was carried out in the same manner as in Test Example 7 except that distilled water was added at the start of the transesterification reaction so that the water content in the reaction system was set to 4.9%, yielding Sample G.

Test Example 10

A transesterification reaction was carried out in the same manner as in Test Example 7 except that distilled water was added at the start of the transesterification reaction so that the water content in the reaction system was set to 9.6%, yielding Sample H.
Table 3 shows the water content in the reaction system and the glyceride composition of the reaction product obtained of each of Test Examples 7 to 10.
[Table 3]

EP 2 287 325 A1

| [Influence of water content in reaction system] | | | | | | | | | | |
|---|---|---|---|---|---|---|---|---|---|---|
| | Reaction product | raw material | Water content Reaction in reaction system [% by mass] | Fatty acid [% by mass] | GLY [% by mass] | MAG [% by [% by mass] | DAG mass] | TAG [% by mass] | DAG+TAG Content [% by mass] | DAG Purity [% by mass] |
| Test Example 7 | Sample E | Material A | 0.2 | 3.4 | 3.3 | 38.6 | 53.9 | 0.8 | 54.7 | 98.5 |
| Test Example 8 | Sample F | Material A | 1.1 | 8.8 | 2.3 | 30.3 | 50.2 | 8.4 | 58.6 | 85.7 |
| Test Example 9 | Sample G | Material A | 4.9 | 28.1 | 1.3 | 20.4 | 39.6 | 10.6 | 50.2 | 79.0 |
| Test Example 10 | Sample H | Material A | 9.6 | 43.3 | 0.8 | 14.7 | 31.4 | 9.7 | 41.1 | 76.4 |

[Influence of glyceride composition in raw material fat or oil]

Test Example 11

A transesterification reaction was carried out in the same manner as in Test Example 7 except that the reaction raw material of the transesterification reaction was changed to Material B, yielding Sample I.

Test Example 12

A transesterification reaction was carried out in the same manner as in Test Example 7 except that the reaction raw material of the transesterification reaction was changed to Material C, yielding Sample J.

Test Example 13

A transesterification reaction was carried out in the same manner as in Test Example 7 except that the reaction raw material of the transesterification reaction was changed to Material D, yielding Sample K.

Test Example 14

A transesterification reaction was carried out in the same manner as in Test Example 7 except that the reaction raw material of the transesterification reaction was changed to Material E, yielding Sample L.

Test Example 15

A transesterification reaction was carried out in the same manner as in Test Example 7 except that the reaction raw material of the transesterification reaction was changed to Material F, yielding Sample M.

Test Example 16

A transesterification reaction was carried out in the same manner as in Test Example 7 except that the reaction raw material of the transesterification reaction was changed to Material G, yielding Sample N.

Test Example 17

A transesterification reaction was carried out in the same manner as in Test Example 7 except that the reaction raw material of the transesterification reaction was changed to Material H, yielding Sample O.
Table 4 shows the water content in the reaction system and the glyceride composition of the reaction product obtained of each of Test Examples 11 to 17.
[Table 4]

| | Reaction product | Reaction raw material | Water content in reaction system [% by mass] | Fatty acid [% by mass] | GLY [% by mass] | MAG [% by mass] | DAG [% by mass] | TAG [% by mass] | DAG+TAG Content [% by mass] | DAG Purity [% by mass] |
|---|---|---|---|---|---|---|---|---|---|---|
| [Influence of glyceride composition in raw material fat or oil] | | | | | | | | | | |
| Test Example 11 | Sample I | Material B | 0.2 | 2.5 | 2.7 | 34.4 | 51.7 | 8.6 | 60.4 | 85.7 |
| Test Example 12 | Sample J | Material | 0.2 | 2.4 | 2.0 | 29.1 | 48.7 | 17.8 | 66.5 | 73.2 |
| Test Example 13 | Sample K | Material D | 0.2 | 2.2 | 1.2 | 21.6 | 43.6 | 31.4 | 75.0 | 58.2 |
| Test Example 14 | Sample L | Material E | 0.2 | 2.0 | 0.7 | 15.4 | 39.0 | 42.8 | 81.8 | 47.6 |
| Test Example 15 | Sample M | Material F | 0.2 | 3.3 | 2.8 | 35.0 | 54.9 | 3.9 | 58.8 | 93.3 |
| Test Example 16 | Sample N | Material G | 0.2 | 3.8 | 1.6 | 26.1 | 51.7 | 16.9 | 68.5 | 75.4 |
| Test Example 17 | Sample O | Material H | 0.2 | 3.4 | 1.1 | 20.5 | 48.4 | 26.7 | 75.0 | 64.5 |

[Influence of lipase concentration]

Test Example 18

250 g of Material Awere loaded into a 500-ML four-necked flask equipped with a stirring blade (75 mm × 20 mm). The flask was left to stand still at 50°C for 30 minutes to stabilize Material A in the flask at 50°C. Next, while Material A was being stirred under the condition of a temperature of 50°C and a stirring of 300 r/min, an immobilized lipase, Lipozyme RM IM (water content: 5.0%), was added at 5% (12.5 g) with respect to Material A, and a transesterification reaction was started. Nitrogen sealing was performed immediately, to thereby convert to a nitrogen atmosphere. After the transesterification reaction was carried out for 8 hours, the immobilized lipase, Lipozyme RM IM, was filtrated off, yielding Sample P.

Test Example 19

A transesterification reaction was carried out in the same manner as in Test Example 18 except that the immobilized lipase, Lipozyme RM IM, (water content: 5.0%) to be used for the transesterification reaction was added at 10% (25.0 g) with respect to Material A, yielding Sample Q.

Test Example 20

A transesterification reaction was carried out in the same manner as in Test Example 18 except that the immobilized lipase, Lipozyme RM IM, (water content: 5.0%) to be used for the transesterification reaction was added at 20% (50.0 g) with respect to Material A, yielding Sample R.

Test Example 21

90 g of Material I were loaded into a 200-ML four-necked flask equipped with a stirring blade (50 mm × 18 mm). The flask was left to stand still at 50°C for 30 minutes to stabilize Material I in the flask at 50°C. Next, while Material I was being stirred under the condition of a temperature of 50°C and a stirring of 300 r/min, an immobilized lipase, Lipozyme RM IM (water content: 2.6%), was added at 1% (0.9 g) with respect to Material I, and a transesterification reaction was started. Nitrogen sealing was performed immediately, to thereby convert to a nitrogen atmosphere. After the transesterification reaction was carried out for 24 hours, the immobilized lipase, Lipozyme RM IM, was filtrated off, yielding Sample W. The content of trans-unsaturated fatty acids in Sample W was 0.2%.

Test Example 22

A transesterification reaction was carried out in the same manner as in Test Example 21 except that the immobilized lipase, Lipozyme RM IM, (water content: 2.6%) to be used for the transesterification reaction was added at 2% (1.8 g) with respect to Material I. The transesterification reaction was carried out for 5 hours, and then the immobilized lipase, Lipozyme RM IM, was filtrated off, yielding Sample X. The content of trans-unsaturated fatty acids in Sample X was 0.2%.

Test Example 23

A transesterification reaction was carried out in the same manner as in Test Example 21 except that the immobilized lipase, Lipozyme RM IM, (water content: 2.6%) to be used for the transesterification reaction was added at 5% (4.5 g) with respect to Material I. The transesterification reaction was carried out for 3 hours, and then the immobilized lipase, Lipozyme RM IM, was filtrated off, yielding Sample Y. The content of trans-unsaturated fatty acids in Sample Y was 0.2%.

Test Example 24

A transesterification reaction was carried out in the same manner as in Test Example 21 except that the immobilized lipase, Lipozyme RM IM, (water content: 2.6%) to be used for the transesterification reaction was added at 20% (18.0 g) with respect to Material I. The transesterification reaction was carried out for 1 hour, and then the immobilized lipase, Lipozyme RM IM, was filtrated off, yielding Sample Z. The content of trans-unsaturated fatty acids in Sample Z was 0.2%.

Table 5 shows the water content in the reaction system and the glyceride composition of the reaction product obtained of each of Test Examples 18 to 24.

[Table 5]

| | Reaction product | Reaction raw material | Water content in reaction system [% by mass] | Fatty acid [% by mass] | GLY [% by mass] | MAG [% by mass] | DAG [% by mass] | TAG [% by mass] | DAG+TAG Content [% by mass] | DAG Purity [% by mass] |
|---|---|---|---|---|---|---|---|---|---|---|
| [Influence of lipase concentration] | | | | | | | | | | |
| Test Example 18 | Sample P | Material A | 0.3 | 4.7 | 3.9 | 63.3 | 28.0 | 0.1 | 28.1 | 99.8 |
| Test Example 19 | Sample Q | Material A | 0.6 | 5.0 | 4.5 | 46.1 | 44.3 | 0.1 | 44.4 | 99.8 |
| Test Example 20 | Sample R | Material A | 1.0 | 6.4 | 3.5 | 39.7 | 50.3 | 0.2 | 50.4 | 99.7 |
| Test Example 21 | Sample W | Material I | 0.04 | 2.5 | 2.4 | 31.8 | 53.2 | 10.1 | 63.3 | 84.1 |
| Test Example 22 | Sample X | Material I | 0.1 | 2.1 | 3.0 | 36.0 | 50.2 | 8.6 | 58.9 | 85.4 |
| Test Example 23 | Sample Y | Material I | 0.1 | 3.6 | 2.1 | 29.5 | 52.7 | 12.1 | 64.8 | 81.4 |
| Test Example 24 | Sample Z | Material I | 0.4 | 4.3 | 1.8 | 26.5 | 52.2 | 15.2 | 67.4 | 77.4 |

[Influence of lipase form]

Test Example 25

100 g of Material A were loaded into a 200-ML four-necked flask equipped with a stirring blade (75 mm × 20 mm) . The flask was left to stand still at 50°C for 30 minutes to stabilize Material A in the flask at 50°C. Next, while Material A was being stirred under the condition of a temperature of 50°C and a stirring of 300 r/min, Lipase OF as a non-immobilized lipase (Meito Sangyo Co. , Ltd. , water content: 6.5%) was added at 1% (1.0 g) with respect to Material A together with 0.5 g of distilled water, and a transesterification reaction was started. Nitrogen sealing was performed immediately, to thereby convert to a nitrogen atmosphere. When the transesterification reaction was carried out for 48 hours, the glyceride composition reached equilibrium. Then, the lipase was precipitated by 10-minute centrifugation at 3000 r/min, yielding Sample S.

Test Example 26

100 g of Material A were loaded into a 200-ML four-necked flask equipped with a stirring blade (75 mm × 20 mm) . The flask was left to stand still at 50°C for 30 minutes to stabilize Material A in the flask at 50°C. Next, while Material A was being stirred under the condition of a temperature of 50°C and a stirring of 300 r/min, Palatase as a non-immobilized lipase (Novozymes Japan Ltd., water content: 51%) was added at 1% (1.0 g) with respect to Material A, and a transesterification reaction was started. Nitrogen sealing was performed immediately, to thereby convert to a nitrogen atmosphere. When the transesterification reaction was carried out for 48 hours, the glyceride composition reached equilibrium. Then, the lipase was precipitated by 10-minute centrifugation at 3000 r/min, yielding Sample T.
Table 6 shows the water content in the reaction system and the glyceride composition of the reaction product obtained of each of Test Examples 25 and 26.
[Table 6]

| [Influence of lipase form] | | | | | | | | | | |
|---|---|---|---|---|---|---|---|---|---|---|
| | Reaction product | Reaction raw material | Water content in reaction system by mass] | Fatty acid [% by mass] | GLY [% by mass] | MAG [% by mass] | DAG [% by mass] | TAG [% by mass] | DAG+TAG Content [% by mass] | DAG Purity [% by mass] |
| Test Example 25 | Sample S | Material A | 0.6 | 9.5 | 5.2 | 57.6 | 27.3 | 0.4 | 27.7 | 98.4 |
| Test Example 26 | Sample T | Material A | 0.6 | 3.7 | 3.9 | 64.1 | 28.2 | 0.0 | 28.3 3 | 99.8 |

[Influence of glycerin content in raw material]

Test Example 27

90 g of Material J were loaded into a 200-ML four-necked flask equipped with a stirring blade (50 mm × 18 mm) . The flask was left to stand still at 50°C for 30 minutes to stabilize Material J in the flask at 50°C. Next, while Material J was being stirred under the condition of a temperature of 50°C and a stirring of 300 r/min, an immobilized lipase, Lipozyme RM IM (water content: 3.2%), was added at 5% (4.5 g) with respect to Material J, and a transesterification reaction was started. Nitrogen sealing was performed immediately, to thereby convert to a nitrogen atmosphere. After the transesterification reaction was carried out for 1. 5 hours, the immobilized Lipase, Lipozyme RM IM, was filtrated off, yielding Sample AA.

Test Example 28

A transesterification reaction was carried out for 4 hours in the same manner as in Test Example 27 except that the reaction raw material of the transesterification reaction was changed to a mixture of 87.3 g of Material J and 2.7 g of glycerin (glycerin content in reaction raw material: 3%), yielding Sample AB.

Test Example 29

A transesterification reaction was carried out for 4 hours in the same manner as in Test Example 27 except that the reaction raw material of the transesterification reaction was changed to a mixture of 85.5 g of Material J and 4.5 g of glycerin (glycerin content in reaction raw material: 5%), yielding Sample AC.

Test Example 30

A transesterification reaction was carried out for 4 hours in the same manner as in Test Example 27 except that the reaction raw material of the transesterification reaction was changed to a mixture of 83.7 g of Material J and 6.3 g of glycerin (glycerin content in reaction raw material.: 7%), yielding Sample AD.

Test Example 31

A transesterification reaction was carried out for 4 hours in the same manner as in Test Example 27 except that the reaction raw material of the transesterification reaction was changed to a mixture of 81.9 g of Material J and 8.1 g of glycerin (glycerin content in a reaction raw material: 9%), yielding Sample AE.

Test Example 32

A transesterification reaction was carried out for 4 hours in the same manner as in Test Example 27 except that the reaction raw material of the transesterification reaction was changed to a mixture of 80.1 g of Material J and 9.9 g of glycerin (glycerin content in a reaction raw material: 11%), yielding Sample AF.
Table 7 shows the water content in the reaction system and the glyceride composition of the reaction product obtained of each of Test Examples 27 to 32.
[Table 7]

| [Influence of glycerin content in raw material] | | | | | | | | | | |
|---|---|---|---|---|---|---|---|---|---|---|
| | Reaction product | Reaction raw material | Water content in reaction system [% by mass] | Fatty acid [% by mass] | GLY [%by mass] | MAG [% by mass] | DAG % by mass] | TAG [% by mass] | DAG+TAG Content [% by mass] | DAG Purity [% by mass] |
| Test Example 27 | Sample AA | Material J | 0.2, | 1.2 | 2.4 | 32.3 | 54.3 | 9.7 | 64.0 | 84.8 |
| Test Example 28 | Sample AB | MaterialJ +glycerin (glycerin. 3%) | 0.2 | 1.0 | 3.5 | 37.1 | 50.0 | 8.4 | 58.4 | 85.6 |
| Test Example 29 | Sample AC | Material J +glycerin (glycerin. 5%) | 0.2 | 0.9 | 4.4 | 42.2 | 44.8 | 7.8 | 52.5 | 85.2 |
| Test Example 30 | Sample AD | Material J +glycerin (glycerin 7%) | 0.2 | 0.7 | 5.4 | 47.0 | 40.1 | 6.9 | 47.0 | 85.4 |
| Test Example 31 | Sample AE | Material J +glycerin (glycerin. 9%) | 0.2 | 0.5 | 6.2 | 48.6 | 38.3 | 6.4 | 44.7 | 85.6 |
| Test Example 32 | Sample AF | Material J +glycerin (glycerin. 11%) | 0.2 | 0.5 | 6.2 | 47.8 | 39.1 | 6.3 | 45.4 | 86.0 |

[Influence of deodorization condition]

Test Examples 33 to 36

[Enzymatic transesterification reaction treatment]

1000 g of Material J were loaded into a 2-L four-necked flask equipped with a stirring blade (90 mm × 24 mm). The flask was left to stand still at 50°C for 30 minutes to stabilize Material J in the flask at 50°C. Next, while Material J was being stirred under the condition of a temperature of 50°C and a stirring of 300 r/min, a commercially available immobilized lipase, Lipozyme RM IM (water content: 3.2 %), was added at 5% (50 g) with respect to Material J, and a transesterification reaction was started. Nitrogen sealing was performed immediately, to thereby convert to a nitrogen atmosphere . After the transesterif icat ion reaction was carried out for 3 hours, the immobilized lipase was filtrated off, yielding a reaction product.

[Distillation treatment]

The reaction product was distilled under the operation conditions of a heater temperature of 235°C, a pressure of 1.5 Pa, and a flow rate of 150 ml/h by using a Wiped Film Evaporating Apparatus (Shinko Pantec Co., Ltd. , Model2-03, internal diameter: 5 cm, heat transfer area: 0.03 m$^2$), yielding a distillate oil. The distillate oil accounted for 61% of the reaction product and a distillation recovery oil accounted for 39%. Table 1 shows the glyceride composition of the distillation recovery oil.

[Acid treatment]

A 10% aqueous solution of citric acid was added to the distillate oil at 2%, the mixture was mixed at a temperature of 70°C for 30 minutes at 400 r/min, and then the mixture was subj ected to dehydration under reduced pressure for 30 minutes while being mixed at a temperature of 100°C at a degree of vacuum of 400 Pa at 400 r/min, yielding an acid-treated oil.

[Water washing treatment]

Distilled water warmed to a temperature of 70°C was added to the acid-treated oil at 10%, and the mixture was vigorously mixed at a temperature of 70°C for 30 minutes at 600 r/min and then centrifuged to separate an oil phase. This water washing operation was carried out three times, and dehydration under reduced pressure was performed at a temperature of 100°C at a degree of vacuum of 400 Pa for 30 minutes, yielding a water-washed oil.

[Deodorization treatment]

Deodorization treatment was performed in a batch mode. A rotaryvacuumpump, TYPE 160VP-DCuteVac, manufactured by Hitachi, Ltd. was used as a vacuum pump. 100 g of the water-washed oil were introduced into a 300-ML glass Claisen's flask, and then a steam generating apparatus was connected to the 300-ML glass Claisen's flask with a capillary glass tube having an internal diameter of 2.5 mm. Nitrogen was passed through the inside of the apparatus while being bubbled at a flow rate of 1 L/min at a temperature of 70°C for 10 minutes, and thus the inside of the apparatus was completely substituted with nitrogen. The inside of the flask was brought to a vacuum state with the vacuum pump, and the system was heated with a mantle heater. It took 6 to 8 minutes to heat from the temperature of 70°C to the temperature of 200°C, and it took 2 to 6 minutes to heat from the temperature of 200°C to the temperature of 245°C. The deodorization temperature, the deodorization time, and the amount of steam were set at the respective conditions shown in Table 8, and the pressure was set at 0.2 to 0 .4 kPa. After completion of the deodorization, the mantle heater was removed, and the system was cooled with a cold air blower, over 1 to 2 minutes to cool from the deodorization temperature to the temperature of 200°C, and over 5 to 7 minutes to cool from the temperature of 200°C to the temperature of 70°C. After the system had been cooled to the temperature of 70°C, nitrogen was blown into the deodorization apparatus to return the system to normal pressure. Tocopherol (Riken E oil 600: Riken Vitamin Co., Ltd.) was added at 200 ppm with respect to the water-washed oil, yielding deodorized oils of Test Examples 33 to 36. Table 8 shows physical properties of the deodorized oils of Test Examples 33 to 36.

[Sensory evaluation]

The evaluation of the deodorized oils of Test Examples 33 to 36 on the flavor (rich body taste, sensation of stimulation and heaviness) was performed by five panels. Each member ate 0.5 to 5 g each of the deodorized oils, and performed a sensory evaluation according to the criteria shown in Table 9 below. The results are shown in Table 8. The term "rich body taste" as used herein refers to a favorable flavor that is characteristic to the diacylglycerols produced by the production process of the present invention, and to a flavor in the state where umami or the like spreads in the mouth and richness with well-balanced palatability is present. Further, the terms "sensation of stimulation" and "heaviness" refer to a flavor attributable to an undeodorized fat or oil as a raw material or an unfavorable flavor coming from impurities produced in the process of producing a diacylglycerol-rich fat or oil, and to a stimulating sensation occurring in the mouth or the throat (sensation of stimulation) and the sensation in the mouth that feels like sticking viscously (heaviness).

[Table 8]

| [Influence of deodorization condition] | | | | | | | | |
|---|---|---|---|---|---|---|---|---|
| | Deodorization condition | | | | | | Evaluation on flavor | |
| | Temperature [°C] | Time (minute) | Amount of steam [% by mass ] | Color [10R+Y] | DAG content [% by mass] | Trans fat content [% by mass] | Rich body taste | sensation of stimulation and heaviness |
| Test Example 33 | 245 | 8 | 0.7 | 17 | 86.2 | 0.5 | 4 | 1 |
| Test Example 34 | 245 | 20 | 2 | 18 | 86.1 | 0.8 | 4 | 3 |
| Test Example 35 | 245 | 45 | 3 | 18 | 85.8 | 1.3 | 4 | 4 |
| Test Example 36 | 245 | 60 | 5 | 18 | 85.8 | 1.6 | 2 | 4 |
| Test Example 37 | 245 | 45 | 4 | 17 | 88.1 | 1.2 | 4 | 4 |

[Table 9]

| [Criteria for evaluation on flavor] | | |
|---|---|---|
| | Rich body taste | Sensation of stimulation and heaviness |
| 5 | Rich body taste is very good. | Sensation of stimulation and heaviness are absent and the sensation is very good. |
| 4 | Rich body taste is good. | Sensation of stimulation and heaviness are absent and the sensation is good. |
| 3 | Rich body taste is slightly present. | Sensation of stimulation and heaviness are absent. |
| 2 | Rich body taste is short. | Sensation of stimulation and/or heaviness are/is slightly present. |
| 1 | Rich body taste lacks. | Sensation of stimulation and/or heaviness are/is present. |

[Recycle of distillation recovery oil]

Test Example 37

280 g (39% by weight) of the distillation recovery oil and 438 g (61% by weight) of Material J, both shown in Table 1 described above, were loaded into a 1-L four-necked flask equipped with a stirring blade (90 mm $\times$ 24 mm) . The flask was left to stand still at 50°C for 30 minutes to stabilize Material J in the flask at 50°C. Next, while the distillation recovery oil and Material J were being stirred under the condition of a temperature of 50 °C and a stirring of 300 r/min, a commercially available immobilized lipase, Lipozyme RM IM (water content: 3.2%), was added at 10% (71.8 g) with respect to the total amount of the distillation recovery oil and Material J, and a transesterification reaction was started- Nitrogen sealing was performed immediately, to thereby convert to a nitrogen atmosphere. After the transesterification reaction was carried out for 5 hours, the immobilized lipase was filtrated off, yielding a reaction product. Next, the distillation treatment and the subsequent steps were carried out in the same conditions as in Test Example 35, yielding a deodorized oil of Test Example 37. The deodorized oil was evaluated for the flavor (rich body taste, sensation of stimulation and heaviness) in accordance with the above-mentioned [Criteria for evaluation on flavor].
Table 8 shows the results of evaluation on physical properties and flavor of the deodorized oil of Test Example 37.
As evident from Tables 2 to 8, it was found that when monoacylglycerols were subjected to a transesterification reaction using a lipase in the presence of water, a fat or oil composition rich in diacylglycerols was efficiently obtained. As evident from Table 2, it was found that when monoacylglycerols were subjected to an transesterification reaction in a hexane solvent using a lipase in the presence of water, a fat or oil composition rich in diacylglycerols was obtained (Test Example 4) . In addition, it was found that when a reacted oil obtained by subj ecting an undeodorized fat or oil and glycerin to a glycerolysis reaction was used as raw material monoacylglycerols for an transesterification reaction, a diacylglycerol-rich fat or oil having a small content of trans-unsaturated fatty acids was obtained (Test Examples 5 and 6) .
From Table 3, it was found that when the water content in a reaction system became large, the concentration of fatty acids in a reaction product increased, and hence the water content was preferably small in order to obtain a fat or oil composition rich in diacylglycerols, and the water content was preferably 4% or less in order to obtain a fat or oil composition having a purity of diacylglycerols of 80% or more (Test Examples 7 to 10).
From Table 4, it was found that when the content of triacylglycerols and the content of diacylglycerols in a reaction raw material became large, the total content of diacylglycerols and triacylglycerols in a reaction product increased, but the total content of diacylglycerols and triacylglycerols in the reaction raw material was preferably smaller from the viewpoint of the purity of diacylglycerols (Test Examples 11 to 17).
From Table 5, it was also found that a lipase concentration was preferably higher from the viewpoint of reducing a reaction time (Test Examples 18 to 24) . Further, from Table 6, it was found that even if a non-immobilized lipase was used, a fat or oil composition having a high purity of diacylglycerols was obtained, though the total content of diacylglycerols and triacylglycerols in a reaction product tended to decline (Test Examples 25 and 26). From Table 7, it was found that when the glycerin content in a reaction raw material was reduced, a fat or oil composition rich in diacylglycerols was obtained (Test Examples 27 to 32).
From Table 8, it was found that when a glycerolysis reacted oil obtained by a glycerolysis reaction between an undeodorized fat or oil and glycerin was used to perform a transesterification reaction, and then a deodorization treatment was carried out so that a thermal history fell within a given range, there was efficiently obtained a diacylglycerol-rich fat or oil which was low in the content of trans-unsaturated fatty acids and was good in color and flavor characteristic to diacylglycerols, such as ""umami" and "rich body taste" (Test Examples 33 to 37). Further, it was found that a diacylglycerol-rich fat or oil having good flavor could also be obtained by using a fat or oil recovered in a distillation step after a transesterification reaction as raw material monoacylglycerols for a transesterification reaction.

**Claims**

1. A process for producing a diacylglycerol-rich fat or oil, comprising subjecting monoacylglycerols to a transesterification reaction by using a lipase in the presence of water.

2. The process for producing a diacylglycerol-rich fat or oil according to claim 1, wherein 4% by mass or less of water is contained in a reaction system.

3. The process for producing a diacylglycerol-rich fat or oil according to claim 1 or 2, wherein the content of triacylglycerols in the monoacylglycerols is 50% by mass or less.

**4.** The process for producing a diacylglycerol-rich fat or oil according to any one of claims 1 to 3, wherein the monoacylglycerols comprise a glycerolysis reacted oil obtained by a glycerolysis reaction between a fat or oil and glycerin.

**5.** The process for producing a diacylglycerol-rich fat or oil according to any one of claims 1 to 4, wherein the content of glycerin in the monoacylglycerols is 15% by mass or less.

**6.** The process for producing a diacylglycerol-rich fat or oil according to any one of claims 1 to 5, wherein the content of trans-unsaturated fatty acids in the constituent fatty acids of the monoacylglycerols is 2% by mass or less.

**7.** The process for producing a diacylglycerol-rich fat or oil according to any one of claims 1 to 6, wherein the purity of diacylglycerols in the diacylglycerol-rich fat or oil [diacylglycerols/ (diacylglycerols + triacylglycerols) $\times$ 100] is 50% by mass or more.

**8.** The process for producing a diacylglycerol-rich fat or oil according to any one of claims 1 to 7, further comprising carrying out, after the transesterification reaction, a deodorization treatment in a range in which a relationship between a deodorization time (x) and a deodorization temperature (y) satisfies the following equation (i):

$$(i) \quad 350 \leq (y-210) \times x \leq 2100$$

(provided that $215 \leq y \leq 280$) wherein x represents the deodorization time (minute) and y represents the deodorization temperature (°C) .

**9.** The process for producing a diacylglycerol-rich fat or oil according to any one of claims 1 to 8, wherein a distillation recovery oil recovered during a distillation step of the diacylglycerol-rich fat or oil is used as a part or a whole of the monoacylglycerols.

<div align="center">

**INTERNATIONAL SEARCH REPORT**

</div>

| | International application No. |
|---|---|
| | PCT/JP2009/000513 |

| A. CLASSIFICATION OF SUBJECT MATTER |
|---|
| *C12P7/64(2006.01)i* |
| According to International Patent Classification (IPC) or to both national classification and IPC |

| B. FIELDS SEARCHED |
|---|
| Minimum documentation searched (classification system followed by classification symbols) |
| C12P7/64 |
| Documentation searched other than minimum documentation to the extent that such documents are included in the fields searched |
| Electronic data base consulted during the international search (name of data base and, where practicable, search terms used) |
| CA/CAPLUS/BIOSIS/MEDLINE/WPIDS(STN), JSTPlus/JST7580(JDreamII), G-Search |

| C. DOCUMENTS CONSIDERED TO BE RELEVANT | | |
|---|---|---|
| Category* | Citation of document, with indication, where appropriate, of the relevant passages | Relevant to claim No. |
| X<br>Y | JP 08-294394 A (Kao Corp.),<br>12 November, 1996 (12.11.96),<br>Full text; Par. Nos. [0005] to [0008]<br>& JP 3072022 B2 | 1-3,5,7,9<br>4,6,8 |
| Y<br>A | WO 2000/003031 A1 (Kao Corp.),<br>20 January, 2000 (20.01.00),<br>Full text<br>& JP 2000-023688 A     & EP 1094116 A1<br>& US 6337414 B1       & JP 3720194 B2<br>& EP 1094116 B1       & DE 69938304 E<br>& ES 2300148 T3       & DE 69938304 T2 | 4<br>1-3,5-9 |

☒   Further documents are listed in the continuation of Box C.      ☐   See patent family annex.

| *   Special categories of cited documents: | "T"   later document published after the international filing date or priority date and not in conflict with the application but cited to understand the principle or theory underlying the invention |
|---|---|
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| 15 April, 2009 (15.04.09) | 28 April, 2009 (28.04.09) |
| Name and mailing address of the ISA/<br>    Japanese Patent Office | Authorized officer |
| Facsimile No. | Telephone No. |

Form PCT/ISA/210 (second sheet) (April 2007)

<table>
<tr>
<td colspan="2" align="center">**INTERNATIONAL SEARCH REPORT**</td>
<td>International application No.<br>PCT/JP2009/000513</td>
</tr>
</table>

C (Continuation).    DOCUMENTS CONSIDERED TO BE RELEVANT

| Category* | Citation of document, with indication, where appropriate, of the relevant passages | Relevant to claim No. |
|---|---|---|
| Y<br>A | JP 2006-328383 A  (Kao Corp.),<br>07 December, 2006 (07.12.06),<br>Full text<br>& EP 1717318 A1          & US 2006/0258872 A1<br>& CN 1854277 A          & KR 2006-113447 A<br>& EP 1717318 B1          & DE 602006001891 E | 6<br>1-5,7-9 |
| Y<br>A | Takeyuki HAMAMOTO et al., "Yushi no Dasshu to Shokuyo Abura no Anteisei", Nippon Yuka Gakkaishi, 1999, Vol.48, No.10, pages 1123 to 1131 | 8<br>1-7,9 |
| Y<br>A | Shizuyuki OTA et al., "Shokuyo Yushi no Gijutsuteki Hensen", Shokuhin Kikai Sochi, 1989, March, Vol.295, pages 73 to 80 | 8<br>1-7,9 |
| Y<br>A | Shizuyuki OTA et al., "Shokuyo Yushi no Gijutsuteki Hensen", Shokuhin Kikai Sochi, 1989, February, Vol.294, pages 70 to 79 | 8<br>1-7,9 |
| Y<br>A | Shizuyuki OTA et al., "Shokuyo Yushi no Gijutsuteki Hensen", Shokuhin Kikai Sochi, 1989, January, Vol.293, pages 76 to 83 | 8<br>1-7,9 |
| A | KRISTENSEN, Janni Brogaard, et al., Process optimization using response surface design and pilot plant production of dietary diacylglycerols by lipase-catalyzed glycerolysis., Journal of agricultural and food chemistry, 2005, Vol.53, No.18, pages 7059-7066 | 1-9 |

Form PCT/ISA/210 (continuation of second sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6065310 A
- JP 6065311 A
- JP 4330289 A
- JP 2000345189 A
- JP 2006137923 A

**Non-patent literature cited in the description**

- Method for preparing fatty acid methyl esters (2. 4. 1. 2 - 1996). Standard Methods for the Analysis of Fats, Oils and Related Materials. 1996